# EUROPÄISCHE PATENTSCHRIFT

(11) **EP 1 968 458 B1**
(45) Veröffentlichungstag und Bekanntmachung des Hinweises auf die Patenterteilung: **11.06.2014**
(21) Anmeldenummer: 06828611.1
(22) Anmeldetag: 05.12.2006
(51) Int. Cl.: A61B 17/16, A61B 17/00

(54) **CHIRURGISCHES BEARBEITUNGSWERKZEUG**
SURGICAL MACHINING TOOL
DISPOSITIF DE TRAITEMENT CHIRURGICAL

(30) Priorität: 05.12.2005 DE 102005058107
(43) Veröffentlichungstag der Anmeldung: 17.09.2008
(73) Patentinhaber: Müller, Erich Johann, 63839 Kleinwallstadt (DE)
(72) Erfinder: Müller, Erich Johann, 63839 Kleinwallstadt (DE)
(74) Vertreter: Becker Kurig Straus
(86) Internationale Anmeldenummer: PCT/DE2006/002160
(87) Internationale Veröffentlichungsnummer: WO 2007/065419

(56) Entgegenhaltungen:
- EP-A1- 0 574 701
- WO-A-01/34039
- WO-A-2004/071310
- US-A- 5 203 653
- US-A- 5 376 092

## Beschreibung

Die Erfindung betrifft ein chirurgisches Bearbeitungswerkzeug zum Herstellen einer Ausnehmung in einem festen Körpergewebe, insbesondere einem Knochen und/oder einem Knorpel nach dem Oberbegriff des Anspruchs 1.

Bekannt sind chirurgische Bearbeitungswerkzeuge, wie beispielsweise in der EP 1 227 762 B1 beschrieben.

Nachteilig ist es, dass die erzeugten ausgearbeiteten Vertiefungen in einigen Fällen im Randbereich einen schnelleren und höheren Abtrag aufweisen als im zentralen Drehzentrum, dem sogenannten Polbereich.

Aufgabe der vorliegenden Erfindung ist es, ein chirurgisches Bearbeitungswerkzeug bereitzustellen, das es ermöglicht, schnell und sicher zu arbeiten und eine vorbestimmte Ausfräsform einzuhalten.

Die Aufgabe wird zum einen gelöst durch ein chirurgisches Bearbeitungswerkzeug zum Herstellen einer Ausnehmung in einem festen Körpergewebe, insbesondere einem Knochen und/oder einem Knorpel, mit einem Fräsbereich, der um eine zentrale Drehachse drehbar ist, mit einer äußeren Mantelfläche des Fräsbereichs und einem durch die Mantelfläche im wesentlichen abgegrenzten, dem zu bearbeitenden Körpergewebe abgewandten Innenbereich, wobei die Mantelfläche im Sinne eines Fräskopfes mit zumindest zwei spanabhebenden Schneidkanten ausgebildet ist, die an der Mantelfläche von einem Drehzentrum zu einem Drehrand des Fräsbereichs verlaufen und insbesondere spiralförmig gekrümmt sind, und zu den Schneidkanten benachbart angeordneten Öffnungen zum Abtransport der Schneidspäne in den Innenbereich, wobei die Schneidkanten mittels Vertiefungen so unterbrochen sind, dass die Schneidkanten aus einzeln schneidend wirkenden Schneidelementen aufgebaut sind, wobei die Mantelfläche zwischen zwei Schneidkanten von zumindest zwei benachbart angeordneten, im wesentlichen parallel zu den Schneidkanten verlaufenden Stoppflächen gebildet ist, die miteinander eine Verschneidungslinie bilden und die das Eindringen der Schneidkanten in das Körpergewebe in einer quer zur Flächenlängsrichtung angeordneten Schneidrichtung bezüglich der Eindringtiefe in das Gewebe begrenzen, wobei die Verschneidungslinie eine erste Stoppfläche begrenzt, die von der Schneidkante abgewendet gebildet ist und ein erstes Eindringen der Schneidkante in das Gewebe begrenzt, und die Verschneidungslinie in Bezug zu einer Schneidkantenspitze eine Differenzhöhe aufweist, die ausgehend von dem Bereich im Drehzentrum zu dem Bereich am Drehrand im wesentlichen konstant bleibt oder in geringem Masse abnimmt und eine zweite Stopfläche, die sich bis an die Öffnung der folgenden Schneidkante erstreckt und das an das erste Eindringen anschließende Eindringen der Schneidkante in das Gewebe begrenzt, zwischen dem sich an der Öffnung befindenden Bereich und der Schneidkantenspitze eine Differenzhöhe aufweist, die ausgehend von dem Bereich im Drehzentrum zu dem Bereich am Drehrand abnimmt.

Die Stopflächen verhindern, dass die Schneidkante zu tief in das Gewebe eindringt. Durch die erste Stopfläche wird die in die Tiefe eindringende Schneidkante abgebremst. Ein weiteres Abbremsen erfolgt dann durch die zweite Stopfläche. Die Höhe der Stopflächen nimmt von dem zentralen Drehzentrum des Fräskopfes bis zum Rand des Fräskopfes ab. Somit wird die Angriffsstärke der Schneidkante zum Rand hin verringert. Der stärkste Angriff erfolgt somit zunächst im Drehzentrum und die gewünschte Fräsform wird nicht verfälscht.

Die Aufgabe wird ebenfalls gelöst durch ein chirurgisches Bearbeitungswerkzeug zum Herstellen einer Ausnehmung in einem festen Körpergewebe, insbesondere einem Knochen und/oder einem Knorpel, insbesondere nach Anspruch 1, mit einem Fräsbereich, der um eine zentrale Drehachse drehbar ist, mit einer äußeren Mantelfläche des Fräsbereichs und einem durch die Mantelfläche im wesentlichen abgegrenzten, dem zu bearbeitenden Körpergewebe abgewandten Innenbereich, wobei die Mantelfläche im Sinne eines Fräskopfes mit zumindest zwei spanabhebenden Schneidkanten ausgebildet ist, die an der Mantelfläche von einem Drehzentrum zu einem Drehrand des Fräsbereichs verlaufen und insbesondere spiralförmig gekrümmt sind, und zu den Schneidkanten benachbart angeordneten Öffnungen zum Abtransport der Schneidspäne in den Innenbereich, wobei die Schneidkanten mittels Vertiefungen so unterbrochen sind, dass die Schneidkanten aus einzeln schneidend wirkenden Schneidelementen aufgebaut sind, mit einer spiralförmig an der Mantelfläche verlaufenden rillenförmigen Vertiefung, die die Schneidkanten an der Mantelfläche im Bereich der Vertiefungen in den Schneidkanten so durchbricht, dass die rillenförmige Vertiefung eine praktisch kontinuierlich fluchtende Fortsetzung der Vertiefungen in den Schneidkanten bildet und eine dadurch spanbrechende Aufteilung der Schneidkanten in Schneidelemente entsteht, wobei die Länge der Schneidelemente ausgehend vom Drehzentrum zum Drehrand abnimmt.

Die rillenförmige Vertiefung stützt den Fräskopf bei dem Vortrieb in das Gewebe und bietet eine Art Führung. Zugleich wird durch die Vertiefung abgespantes Gewebematerial bis zur nächstfolgenden Öffnung transportiert und gelangt von dort durch die Öffnung an die Innenseite des Fräskopfes. Zugleich haben die Vertiefungen in den Schnittkanten eine spanbrechende Wirkung. Die Länge der durch die Vertiefungen gebildeten Schneidelemente nimmt von dem Drehzentrum zum Drehrand ab. Hierdurch wird wiederum eine Verlangsamung des Abtrags vom Drehzentrum zum Drehrand erreicht und die Formsicherheit beim Fräsen erhöht.

Die Aufgabe wird weiterhin gelöst durch ein chirurgisches Bearbeitungswerkzeug zum Herstellen einer Ausnehmung in einem festen Körpergewebe, insbesondere einem Knochen und/oder einem Knorpel, insbesondere nach Anspruch 1 oder 2, mit einem Fräsbereich, der um eine zentrale Drehachse drehbar ist, mit einer äußeren Mantelfläche des Fräsbereichs und einem durch die Mantelfläche im wesentlichen abgegrenzten, dem zu bearbeitenden Körpergewebe abgewandten Innenbereich, wobei die Mantelfläche im Sinne eines Fräskopfes mit zumindest zwei spanabhebenden Schneidkanten ausgebildet ist, die an der Mantelfläche von einem Drehzentrum zu einem Drehrand des Fräsbereichs verlaufen und insbesondere spiralförmig gekrümmt sind, und zu den Schneidkanten benachbart angeordneten Öffnungen zum Abtransport der Schneidspäne in den Innenbereich, wobei die Schneidkanten mittels Vertiefungen so unterbrochen sind, dass die Schneidkanten aus einzeln schneidend wirkenden Schneidelementen aufgebaut sind, wobei die Öffnungen im wesentlichen parallel zu den Schneidkanten durchgehend offen ausgebildet sind und im Bereich des Drehzentrums und im Bereich des Drehrandes enden.

Die großen Öffnungen ermöglichen einen schnellen und zuverlässigen Abtransport der Späne. Die Stabilität des Fräskopfes ist zudem durch die Halterung am Drehzentrum und am Drehrand gegeben.

Vorteilhaft ist es, wenn die Schneidkanten im Querschnitt betrachtet jeweils einen Spanwinkel α aufweisen, der zwischen der Mantelfläche und einer dem Werkstück abgewandten Schneidenfläche gebildet ist, und der von der Schneidkante im Bereich des Drehzentrums zu der Schneidkante im Bereich am Drehrandes zunimmt.

Vorteilhaft ist es, wenn die Schneidkanten im Querschnitt betrachtet einen Freiwinkel γ aufweisen, der zwischen der Mantelfläche und der dem Werkstück zugewandten Schneidfläche ausgebildet ist, und der von der Schneidkante im Bereich des Drehzentrums zu der Schneidkante im Bereich am Drehrandes abnimmt.

Vorteilhaft ist es, wenn der Freiwinkel γ in Abhängigkeit vom Abstand zum Drehzentrum stufenweise und/oder kontinuierlich abnimmt.

Vorteilhaft ist es, wenn die Stopflächen einen konkaven Querschnitt aufweisen.

Vorteilhaft ist es, wenn die dem Werkstück zugewandte Schneidfläche des Werkstücks über die gesamte Schneidkante verlaufend im wesentlichen konstant bleibt.

Vorteilhaft ist es, wenn der Querschnitt der rillenförmigen Ausnehmung ausgerundet oder eckig ist.

Vorteilhaft ist es, wenn die Schneidkante durch Ausnehmungen in etwa 5 bis 8 Schneidelemente unterteilt ist. Vorteilhaft ist es, wenn der Fräsbereich halbkugelförmig oder dreieckförmig oder plan oder kegelförmig oder konisch oder pilzförmig ausgebildet ist.

Vorteilhaft ist es, wenn der Fräsbereich Ausnehmungen in der Seite der Mantelfläche aufweist, um den Fräsbereich an schwer zugängliche Gewebebereiche einzubringen, wobei die Einhüllende des Fräsbereichs praktisch unverändert gegenüber dem Vollwerkzeug bleibt.

Vorteilhaft ist es, wenn der Fräsbereich etwa 2 bis 8, vorzugsweise 6, vorzugsweise mit einem gleichem Abstand über die Mantelfläche angeordnete Schneidkanten aufweist.

Vorteilhaft ist es, wenn der Fräsbereich aus einem hochwarmfesten Metall, insbesondere einer Cr-Co Legierung und/oder hochwarmfesten Kunststoff, insbesondere PEEK (Polyetheretherketon), und/oder keramisches Material, insbesondere mit Zirkonoxid, gebildet ist.

Die Erfindung betrifft wie dargestellt ein chirurgisches Bearbeitungswerkzeug zur Schaffung einer Vertiefung in einem Knorpel- und/oder Knochengewebe für eine Gelenksprothese, insbesondere in einem Hüftgelenksknochen für eine Pfannenprothese, welches eine im wesentlichen halbkugelförmige Mantelfläche und einen dadurch umgrenzten hohlen Innenbereich aufweist, wobei an der Mantelfläche eine sich zum Äquator der Mantelfläche erstreckende Schneide, welche zumindest ein Schneidelement mit einer dem Werkstück zugewandten Schneidefläche und einer dem Werkstück abgewandten Schneidefläche aufweist, und eine damit kooperierende Öffnung in der Mantelfläche zum Abtransport des Spans in den hohlen Innenbereich ausgebildet sind.

Derartige Bearbeitungswerkzeuge weisen jedoch den Nachteil auf, dass der halbkugelförmige Werkzeugkopf zum Rand bzw. zum Äquator hin aufgrund der größeren Schneidegeschwindigkeit einen immer höheren Materialabtrag aufweist. Dadurch weicht die geometrische Form der Vertiefungen von der für die Prothese benötigten Ausgestaltung ab. Folglich kommt es zu einer Passungenauigkeit zwischen der Vertiefung und der darin aufgenommenen Pfannenprothese , so dass sich der Patient in relativ kurzen Abständen einer Operation unterziehen muss, um eine neue Prothese eingesetzt zu bekommen.

Ziel der vorliegenden Erfindung ist es unter anderem, ein chirurgisches Bearbeitungswerkzeug zur Ausbildung einer Vertiefung in einem Knorpel und/oder Knochengewebe, insbesondere in einem Hüftgelenksknochen für eine Pfannenprothese, zu schaffen, durch welches eine Vertiefung mit einer möglichst exakten geometrischen Form herstellbar ist.

Das chirurgische Bearbeitungswerkzeugs weist insbesondere einen Freiwinkel γ des Schneidelements auf, welcher zwischen der Mantelfläche und der dem Werkstück zugewandten Schneidfläche ausgebildet ist, und vom Pol der Mantelfläche in Richtung des Äquators der Mantelfläche abnimmt.

Durch die Abnahme des Freiwinkels γ wird der Materialabtrag zum Rand bzw. Äquator der Halbkugel hin vermindert, so dass die geometrische Form der Vertiefung nahezu exakt der Form des halbkugelförmigen Bearbeitungswerkzeugs entspricht. Aufgrund der exakten Ausbildung der halbkugelförmigen Vertiefung kommt es zu einer besseren Passgenauigkeit zwischen der Pfannenprothese und der Vertiefung, so dass sich die Lebensdauer der eingesetzten Prothese erhöht. Folglich verlängern sich die Intervalle zwischen den Operationen, bei welchen dem Patienten eine neue Prothese eingesetzt werden muss.

Ferner wird durch die Abnahme des Freiwinkels zum Rand des Bearbeitungswerkzeuges hin, die Tendenz des Verlaufens des erfindungsgemäßen Bearbeitungswerkzeuges gegenüber herkömmlichen Bearbeitungswerkzeugen vermindert, da die maximale Schneidleistung des erfindungsgemäßen Bearbeitungswerkzeugen näher am Pol der Mantelfläche angeordnet ist. Dies führt dazu, dass das Bearbeitungswerkzeug gegenüber herkömmlichen Bearbeitungswerkzeugen bei entsprechendem Vorschub in Richtung der Drehachse während des Bearbeitungsvorgangs selbstzentrierend ist.

Durch eine Weiterbildung ist eine Herstellung des Bearbeitungswerkzeuges besonders einfach, insbesondere wenn die Schneide aus einer Vielzahl von Schneideelementen aufgebaut ist.

Mit einer Weiterbildung kann das Schneidverhalten des Bearbeitungswerkzeuges weiterhin verbessert werden, da der Freiwinkel γ in Abhängigkeit vom Abstand zur Rotationsachse kontinuierlich angepasst wird.

Eine Weiterbildung weist den Vorteil auf, dass das Bearbeitungswerkzeug im gesamten Schneidbereich eine wesentliche gleichmäßige Schneideigenschaft aufweist, da die maximale Vordringtiefe hc der Schneide im gesamten Schneidumfang konstant ist, so dass sich die Schneideigenschaften des Bearbeitungswerkzeugs optimieren. Die maximale Vordringtiefe ist hc = 2rπ tan γ, woraus ersichtlich ist, dass eine indirekte Proportionalität zwischen dem Abstand der Schneide zur Drehachse und dem tan γ besteht, wobei der Freiwinkel γ zwischen 0° und 90° liegt, d. h. wenn der Abstand der Schneide zur Drehachse zunimmt, wird der Freiwinkel γ kleiner.

Durch eine Weiterbildung kann das Zerspanvolumen des Werkstoffs gemäß dem Radius angepasst werden, wobei der Freiwinkel γ des Schneidelements und der Spanwinkel α des Schneidelements im Hinblick auf das zu bearbeitende Material in vorteilhafter Weise abgestimmt werden.

Darüber hinaus hat die Weiterbildung den Vorteil, dass sich die effektive Schneidlänge der Schneide erhöht, wobei beim Spanabtrag neben der Scherkraft in Drehrichtung noch eine zusätzliche Komponente der Scherkraft nach außen auftritt, so dass der Materialabtrag mit einem geringeren Kraftaufwand möglich ist.

Eine Weiterbildung weist den Vorteil auf, dass das Bearbeitungswerkzeug während des Bearbeitungsvorgangs dadurch stabilisiert wird, dass mehrere Schneiden gleichzeitig im Eingriff sind, so dass das Bearbeitungswerkzeug kaum von der gewünschten Drehachse abweicht. Ferner werden somit die Vibrationen des Bearbeitungswerkzeugs vermindert, so dass sich die Oberflächenbeschaffenheit der erzeugten Vertiefung verbessert.

Durch eine Weiterbildung kommt es zu einer vorteilhaften Stabilisierung und Vibrationsdämpfung, wobei die Herstellung des Bearbeitungswerkzeugs kostengünstig ist. Ferner sind zumindest drei Schneiden im gleichen Umfang wirksam, um die Stabilität der Konstruktion zu gewährleisten.

Wie die vorstehende Beschreibung zeigt, können vielfache Abwandlungen und Abänderungen von der dargestellten Ausgangsform vorgenommen werden, ohne den Rahmen der Erfindung zu verlassen. So kann beispielsweise die sich vom Pol der Mantelfläche zum Äquator der Mantelfläche erstreckende Schneide auch durch eine Vielzahl von unabhängigen Schneideelementen aufgebaut sein. Ferner kann ein erfindungsgemäßes Bearbeitungswerkzeug fest eingespannt sein, wobei das Werkstück gedreht wird. Darüber hinaus kann die Mantelfläche des Bearbeitungswerkzeugs eine Form aufweisen, die keiner vollkommen Halbkugel entspricht, sondern einer Halbkugel ähnlich ist oder einem Teil einer Halbkugel entspricht, der sich nicht bis zum Äquator erstreckt. Des weiteren kann das Führungselement aus an der Mantelfläche angeordneten Kreiselementen oder einer Vielzahl von einzelnen Segmenten aufgebaut sein.

Weitere Merkmale und Vorteile der Erfindung ergeben sich aus den Ansprüchen und der nachstehenden Beschreibung, in der Ausführungsbeispiele des Gegenstands der Erfindung in Verbindung mit den Zeichnungen näher erläutert sind.

Es zeigen:
Fig. 1 ein Bearbeitungswerkzeug

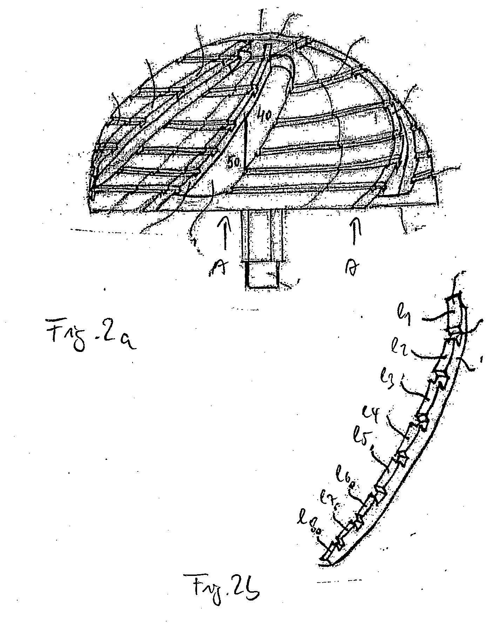

Fig. 2 a eine Seitenansicht eines Bearbeitungswerkzeugs
Fig. 2b eine Schneidkante mit Vertiefungen und Schneidelementen

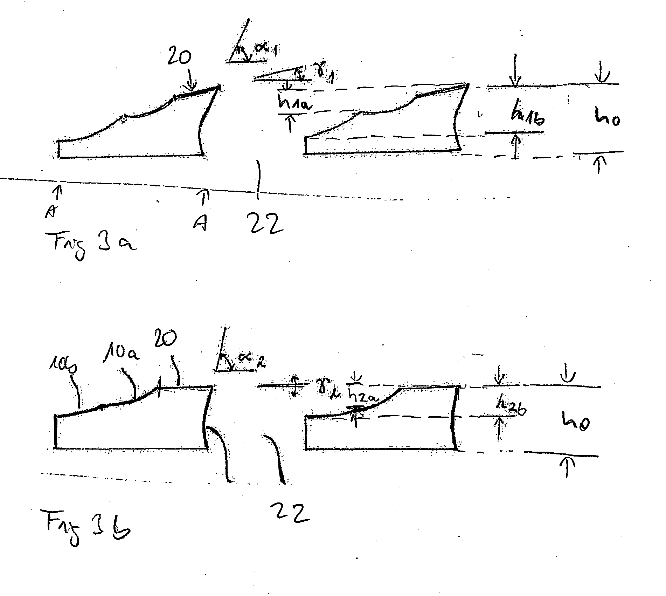

Fig. 3a einen Querschnitt AA durch eine Mantelfläche aus Fig. 2a im Polbereich
Fig. 3b einen Querschnitt AA durch eine Mantelfläche aus Fig. 2a im Äquatorbereich,

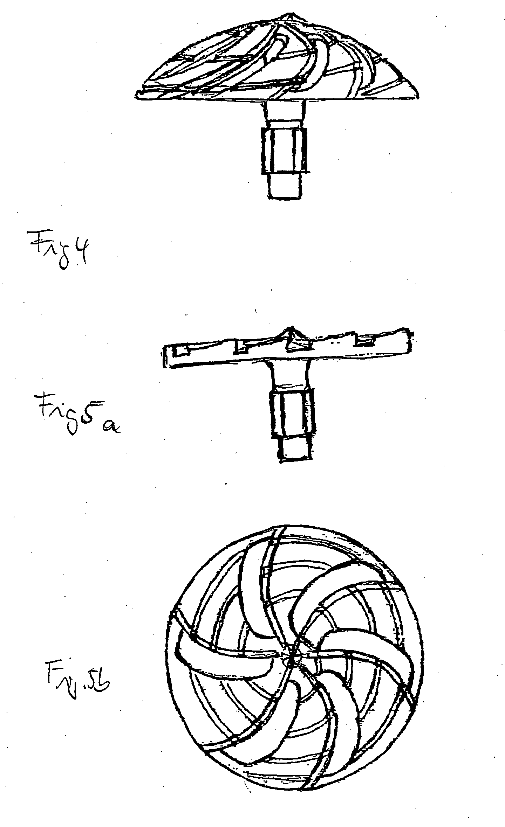

Fig. 4 ein Bearbeitungswerkzeug in Seitenansicht
Fig. 5a ein Bearbeitungswerkzeug in Aufsicht,

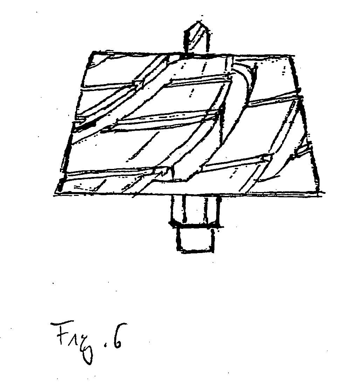

Fig. 6 ein Bearbeitungswerkzeug,

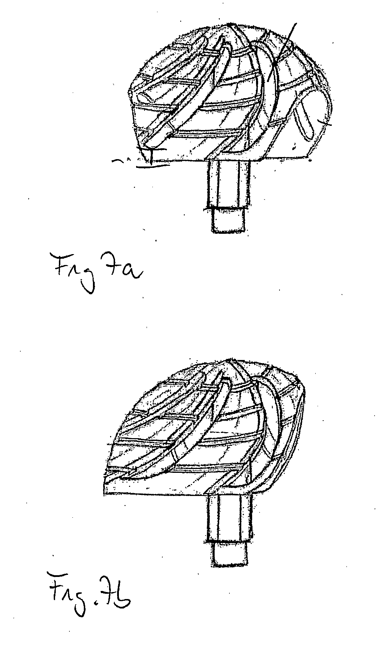

Fig. 7a ein Bearbeitungswerkzeug mit Seitenausnehmung,
Fig. 7b ein Bearbeitungswerkzeug mit Seitenausnehmung,
Fig. 8 ein Bearbeitungswerkzeug mit einem Adapter zum Ansetzen an ein Antriebswerkzeug und Fig. 9 ein Bearbeitungswerkzeug mit einem Adapter zum Ansetzen an ein Antriebswerkzeug.

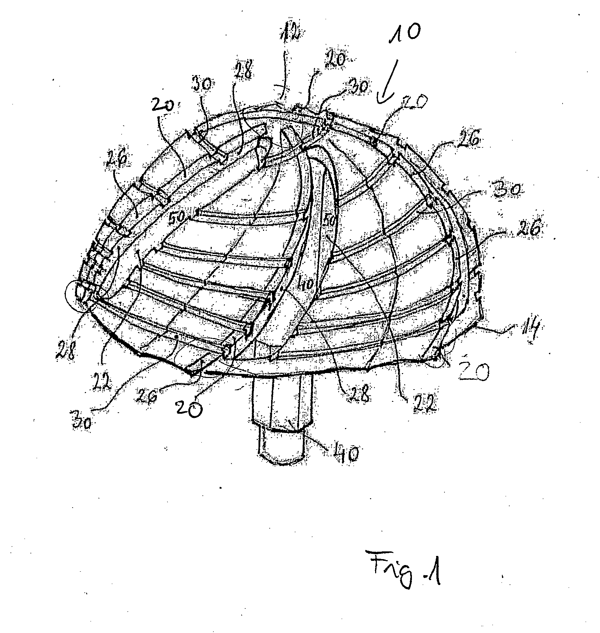

Fig. 1 zeigt ein chirurgisches Bearbeitungswerkzeug mit einer halbkugelförmigen Mantelfläche 10 und einem dadurch umgrenzten Innenbereich 50, das mittels eines Lagerschafts 40 um eine zentrale Achse gedreht werden kann. Die Drehung erfolgt dabei von der Lagerschaftrichtung aus gesehen im Uhrzeigersinn. Vom Drehzentrum, dem Pol 12, aus erstrecken sich Schneidkanten an Schneiden 20, die in einzelne Scheidelemente mittels die Schneidkante durchbrechende Vertiefungen 30 eingeteilt sind. Die Länge der Schneidelemente nimmt vom Pol 12 zum Äquator 14 ab. Die Schneiden weisen eine Schneidfläche 28 auf, die vom Werkstück abgewandt ist und eine Schneidfläche 26, die dem Werkstück zugewandt ist.

Die Vertiefung 30 läuft in Form einer Spirale über die gesamte Mantelfläche vom Pol zum Äquator.

Die neu angebrachte, von Pol zum Äquator spiralförmig verlaufende rillenartige Vertiefung, die eckig oder rund sein kann, führt durch die Schneidkanten. Dies führt zu einzelnen spanbrechenden Schneidelementen, die beim Kreuzen der Rille mit den Schneidekanten entstehen. Die bedeutet eine bessere Schneideigenschaft der Fräse. Weil die Gesamtlänge der Schneidfläche der Schneidkanten durch die Einrichtung der Schneidelemente auf 70% der Schneidkantenlänge reduziert ist, ist die Gefahr des Festfahrens erheblich reduziert. Zugleich ist die Schneideigenschaft nicht vermindert und es wird eine perfekte Knorpel oder Knochenvertiefung erzeugt.

Die Schneidkante wird insgesamt vorzugsweise in etwa 5 bis 8 Schneidelemente unterteilt.

Die Spanbrecher werden zum Äquator durch Rillen mit einem kleineren Abstand zueinander erzeugt. Auf diese Weise nimmt die Länge der Schneidelemente von Pol zum Äquator ständig ab. Dies führt wiederum dazu, dass die Schneidkraft zum Äquator abnimmt und die Führungstreue der Fräse zunimmt.

Zusätzlich wirkt die spiralförmig angelegte Rille selbst als Führungselement, weil der Knorpel/Knochen, der nicht im Spanbrecherbereich vom Schneidelement entfernt wird, in die Spirale eindringt. Dies führt zu einer Stabilisierung der Fräse, bis das nachfolgende Schneidelement sie entfernt. Die Rille dient zudem als Vorschubhilfsmittel, ähnlich einem Korkenzieher, solange bis Druck auf die Fräse ausgeübt wird.

Die Fräse wird an dem Drehzentrum und dem Drehrand zusammengehalten.

Die Spandicke hängt ab von dem Unterschied zwischen der Schneidkante und der benachbarten Mantelfläche, wobei zwischen Mantelfläche und Schneidkante die Öffnung zum Durchführen der Schneidspäne angeordnet ist. Die Arbeitshöhe zwischen Spitze der Schneidkante und Ende der zweiten Mantelfläche (10b) nimmt von dem Schneidkanten bereich im Drehzentrum (h1b) zum Schneidkantenbereich am Drehrand (h2b) vorzugsweise kontinuierlich ab. Dies erhöht die Fräsführungstreue stark.

Die Mantelfläche der Fräse ist zwischen jeweils zwei Schneidkanten von zwei unterschiedlichen Flächen gebildet, eine erste, die von der Schneidkante abgewendet gebildet ist und eine zweite Fläche, die sich bis an die Öffnung zwischen dieser und der benachbarten Schneidkante erstreckt. Die Flächen wirken als Vorschubsstopper.

Der Unterschied zwischen der zweiten Fläche und der folgenden Schneidkante durch einen abnehmenden Abstand zum Äquator führt zu mehr Aggressivität im Polbereich und so zu einer besseren Richtungstreue.

Die Schneidkantenfläche ist gleich breit auf der gesamten Länge vom Pol zum Äquator.

Die Öffnungen in den Mantelflächen, die sich vor jeder Schneidkante befinden, erstrecken sich von einem Abstand von etwa 2 bis 7 mm vom Drehzentrum ("Pol") bis zu einem Abstand von etwa 0,5 bis 3 mm zum Drehrand ("Äquator").

Durch die Erfindung wird eine Vertiefung in ein körpereigenes Hartgewebe, insbesondere Knochen oder Knorpel gearbeitet.

Diese Vertiefung kann insbesondere halbkugelförmig sein und kann dann als Einsatzbereich beispielsweise für Hüftgelenksprothesen dienen.

In einer Ausführungsform eines chirurgischen Bearbeitungswerkzeugs weist es einen halbkugelförmigen Werkzeugkopf 10 in Form einer halbkugelförmigen Mantelfläche 10 und einen dadurch umgrenzten hohlen Innenbereich 50 auf. An der halbkugelförmigen Mantelfläche 10 sind Schneiden 20 angeordnet, welche sich vom Pol 12 der Mantelfläche 10 zum Äquator 14 der Mantelfläche 10 erstrecken, wobei die Schneiden 20 gleichmäßig über der Mantelfläche 10 verteilt sind und spiralförmig gekrümmt sind.

Zum Abtransport des Spans in den hohlen Innenbereich 50 sind Öffnungen 22 in der Mantelfläche 10 zwischen den Schneiden 20 dem Führungselement 30 ausgebildet. Beim Bearbeitungsvorgang dreht sich das dargestellte Bearbeitungswerkzeug, wobei jede Schneide 20 eine Vielzahl von Kerben 24 aufweist, die als Spanbrecher dienen, so dass die von den Schneiden 20 zerteilten Späne eines nicht dargestellten Werkstücks durch die Öffnungen 22 in den hohlen Innenbereich 50 abtransportiert werden, wobei die Länge der Kerben 24 vom Pol 12 zum Äquator 14 zunimmt. Ferner ist ein sich durch den hohlen Innenbereich 50 erstreckender Lagerschaft 40 an der Mantelfläche 10, insbesondere am Pol 12, befestigt, welcher sowohl die Drehachse als auch die Symmetrieachse des Bearbeitungswerkzeugs bildet. Der Lagerschaft 40 ist teilweise durch Öffnungen 22 in dem hohlen Innenbereich 50 zu sehen.

Dargestellt ist eine Seitenansicht der Ausführungsform des Bearbeitungswerkzeugs, wobei insbesondere die Form der halbkugelförmigen Mantelfläche 10 ersichtlich ist.

Beispielsweise wird zwischen der Mantelfläche 10 und der dem Werkstück zugewandten Schneidfläche 26 des Schneidelements bzw. der Schneide 20 ein Freiwinkel γ ausgebildet. Andererseits bildet sich zwischen der Mantelfläche 10 und der dem Werkstück abgewandten Schneidefläche 28 des Schneidelements bzw. der Schneide 20 ein Spanwinkel α.

Darüber hinaus ist der Freiwinkel γ und der Spanwinkel α des Schneidelements 20 im Bereich des Äquators 14 der Mantelfläche 10 dargestellt. Dabei kann der Freiwinkel größer oder kleiner sein, wobei sich der Spanabtrag der Schneide 20 vom Pol 12 der Mantelfläche 10 in Richtung des Äquators 14 der Mantelfläche 10 aufgrund der Abnahme des Freiwinkels γ vermindert.

Wenn der Spanwinkel α größer wird, kann sich das Zerspanvolumen des Werkstoffs gemäß dem Radius anpassen. Somit nimmt der Spanwinkel α der Schneide 20, welcher zwischen Mantelfläche 10 und der dem Werkstück abgewandten Schneidfläche 28 des Schneidelements bzw. der Schneide 20 ausgebildet ist, vorteilhafterweise vom Pol 12 der Mantelfläche 10 in Richtung des Äquators 14 der Mantelfläche 10 zu.

Bei einer graphischen Darstellung können die Schneidleistungen in Bezug auf den Durchmesser von Bearbeitungswerkzeugen gezeigt werden, wobei die x-Achse den Durchmesser von Bearbeitungswerkzeugen und die γ-Achse die Schneidleistung von Bearbeitungswerkzeugen angibt.

Die Schneidleistung des erfindungsgemäßen Bearbeitungswerkzeugs ist gegenüber dem herkömmlichen Bearbeitungswerkzeug größer. Ferner wirken erfindungsgemäße Bearbeitungswerkzeuge bei entsprechendem Vorschub in Richtung der Drehachse während Bearbeitungsvorgangs selbstzentrierend.

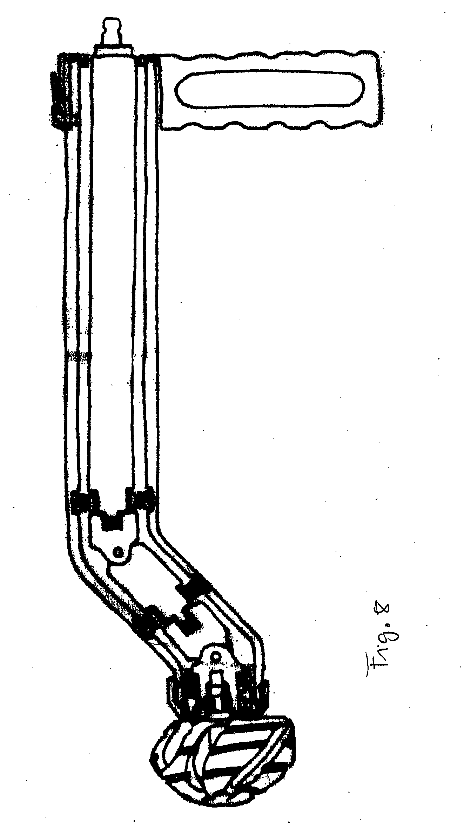

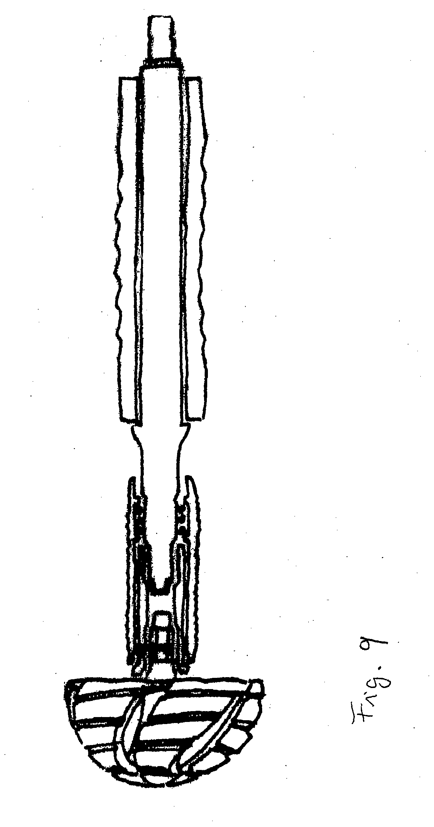

Fig. 8 und Fig. 9 zeigen ein Bearbeitungswerkzeug mit einem Adapter zum Ansetzen an ein Antriebswerkzeug, beispielsweise ein Powertool oder ein Handwerkzeug.

## Patentansprüche

1. Chirurgisches Bearbeitungswerkzeug zum Herstellen einer Ausnehmung in einem festen Körpergewebe, insbesondere einem Knochen und/oder einem Knorpel, mit einem Fräsbereich, der um eine zentrale Drehachse drehbar ist, mit einer äußeren Mantelfläche (10) des Fräsbereichs und einem durch die Mantelfläche im wesentlichen abgegrenzten, dem zu bearbeitenden Körpergewebe abgewandten Innenbereich (50), wobei die Mantelfläche im Sinne eines Fräskopfes mit zumindest zwei spanabhebenden Schneidkanten (20) ausgebildet ist, die an der Mantelfläche (10) von einem Drehzentrum (12) zu einem Drehrand (14) des Fräsbereichs verlaufen und insbesondere spiralförmig gekrümmt sind, und zu den Schneidkanten (20) benachbart angeordneten Öffnungen (22) zum Abtransport der Schneidspäne in den Innenbereich (50), wobei die Schneidkanten (20) mittels Vertiefungen so unterbrochen sind, dass die Schneidkanten aus einzeln schneidend wirkenden Schneidelementen aufgebaut sind,
**dadurch gekennzeichnet, dass** die Mantelfläche (10) zwischen zwei Schneidkanten (20) von zumindest zwei benachbart angeordneten, im wesentlichen parallel zu den Schneidkanten (20) verlaufenden Stopflächen (10a, 10b) gebildet ist, die miteinander eine Verschneidungslinie bilden und die das Eindringen der Schneidkanten (20) bezüglich der Eindringtiefe in das Gewebe begrenzen, wobei die Verschneidungslinie eine erste Stopfläche (10a) begrenzt, die von der Schneidkante abgewendet gebildet ist und ein erstes Eindringen der Schneidkante in das Gewebe begrenzt, und die Verschneidungslinie in Bezug zu einer Schneidkantenspitze eine erste Differenzhöhe (h1a, h2a) aufweist, die ausgehend von dem Bereich im Drehzentrum (h1a) zu dem Bereich am Drehrand (h2a) im wesentlichen konstant bleibt oder in geringem Masse abnimmt und eine zweite Stopfläche (10b), die sich bis an die Öffnung der folgenden Schneidkante erstreckt und das an das erste Eindringen anschließende Eindringen der Schneidkante in das Gewebe begrenzt, zwischen dem sich an der Öffnung befindenden Bereich und der Schneidkantenspitze eine zweite Differenzhöhe (h1b, h2b) aufweist, die ausgehend von dem Bereich im Drehzentrum (h1b) zu dem Bereich am Drehrand (h2b) abnimmt.

2. Chirurgisches Bearbeitungswerkzeug nach Anspruch 1, mit einer spiralförmig an der Mantelfläche (10) verlaufenden rillenförmigen Vertiefung (30), die die Schneidkanten (20) an der Mantelfläche im Bereich der Vertiefungen (24) in den Schneidkanten so durchbricht, dass die rillenförmige Vertiefung (30) eine praktisch kontinuierlich fluchtende Fortsetzung der Vertiefungen (24) in den Schneidkanten bildet und eine dadurch spanbrechende Aufteilung der Schneidkanten (20) in Schneidelemente entsteht, wobei die Länge der Schneidelemente ausgehend vom Drehzentrum (12) zum Drehrand (14) abnimmt.

3. Chirurgisches Bearbeitungswerkzeug nach Anspruch 1 oder 2, wobei die Öffnungen (22) im wesentlichen parallel zu den Schneidkanten (20) durchgehend offen ausgebildet sind und im Bereich des Drehzentrums (12) und im Bereich des Drehrandes (14) enden.

4. Chirurgisches Bearbeitungswerkzeug nach einem der Ansprüche 1 bis 3, **dadurch gekennzeichnet, dass** die Schneidkanten (20) im Querschnitt betrachtet jeweils einen Spanwinkel alpha aufweisen, der zwischen der Mantelfläche (10) und einer dem Werkstück abgewandten Schneidenfläche (28) gebildet ist, und der von der Schneidkante im Bereich des Drehzentrums zu der Schneidkante im Bereich am Drehrandes zunimmt.

5. Chirurgisches Bearbeitungswerkzeug nach einem der Ansprüche 1 bis 4, **dadurch gekennzeichnet, dass** die Schneidkanten (20) im Querschnitt betrachtet einen Freiwinkel gamma aufweisen, der zwischen der Mantelfläche und der dem Werkstück zugewandten Schneidfläche (26) ausgebildet ist, und der von der Schneidkante im Bereich des Drehzentrums zu der Schneidkante im Bereich am Drehrandes abnimmt.

6. Chirurgisches Bearbeitungswerkzeug nach einem der Ansprüche 1 bis 5, **dadurch gekennzeichnet, dass** der Freiwinkel gamma in Abhängigkeit vom Abstand zum Drehzentrum (12) stufenweise und/oder kontinuierlich abnimmt.

7. Chirurgisches Bearbeitungswerkzeug nach einem der Ansprüche 1 bis 6, **dadurch gekennzeichnet, dass** die Stopflächen (10a, 10b) einen konkaven Querschnitt aufweisen.

8. Chirurgisches Bearbeitungswerkzeug nach einem der Ansprüche 1 bis 7, **dadurch gekennzeichnet, dass** die dem Werkstück zugewandte Schneidfläche (26) des Werkzeugs über die gesamte Schneidkante (20) verlaufend im wesentlichen konstant bleibt.

9. Chirurgisches Bearbeitungswerkzeug nach einem der Ansprüche 1 bis 8, **dadurch gekennzeichnet, dass** der Querschnitt der rillenförmigen Ausnehmung (30) ausgerundet oder eckig ist.

10. Chirurgisches Bearbeitungswerkzeug nach einem der Ansprüche 1 bis 9, **dadurch gekennzeichnet, dass** die Schneidkante durch Ausnehmungen (24) in etwa 5 bis 8 Schneidelemente unterteilt ist.

11. Chirurgisches Bearbeitungswerkzeug nach einem der Ansprüche 1 bis 10, **dadurch gekennzeichnet, dass** der Fräsbereich halbkugelförmig oder dreieckförmig oder plan oder kegelförmig oder konisch oder pilzförmig ausgebildet ist.

12. Chirurgisches Bearbeitungswerkzeug nach einem der Ansprüche 1 bis 11, **dadurch gekennzeichnet, dass** der Fräsbereich Ausnehmungen in der Seite der Mantelfläche aufweist, um den Fräsbereich an schwer zugängliche Gewebebereiche einzubringen, wobei die Einhüllende des Fräsbereichs praktisch unverändert gegenüber dem Vollwerkzeug bleibt.

13. Chirurgisches Bearbeitungswerkzeug nach einem der Ansprüche 1 bis 12, **dadurch gekennzeichnet, dass** der Fräsbereich etwa 2 bis 8, vorzugsweise 6, vorzugsweise mit einem gleichem Abstand über die Mantelfläche (10) angeordnete Schneidkanten (20) aufweist.

14. Chirurgisches Bearbeitungswerkzeug nach einem der Ansprüche 1 bis 12, **dadurch gekennzeichnet, dass** der Fräsbereich aus einem hochwarmfesten Metall, insbesondere einer Cr-Co-Legierung gebildet ist.

## Claims

1. Surgical machining tool for making a recess in solid body tissue, particularly in a bone and/or cartilage, with a milling area that is rotatable about a central axis of rotation, with an outer convex surface (10) of the milling area, and with an inner area (50) that is substantially delimited by the convex surface and faces away from the body tissue that is to be machined, wherein the convex surface is constructed with at least two machining cutting edges (20) after the manner of milling head, which cutting edges extend from a centre of rotation (12) to a periphery of rotation (14) on the convex surface (10), and in particular are curved in the form of a spiral, and openings (22) arranged adjacent to the cutting edges (20) for transporting the chips from cutting away into the inner region (50), wherein the cutting edges (20) are interrupted by depressions in such manner that the cutting edges are constructed of cutting elements having individual cutting action,
**characterised in that** the convex surface (10) is formed between two cutting edges (20) of at least two adjacently arranged stop surfaces (10a, 10b) that extend substantially parallel to the cutting edges (20), which stop surfaces form a line of intersection with each other and limit the penetration of the cutting edges (20) with regard to the depth of penetration thereof into the tissue, wherein the line of intersection delimits a first stop surface (10a), which is formed facing away from the cutting edge and limits a first penetration by the cutting edge into the tissue, and the line of intersection has a first height difference (h1a, h2a) relative to a tip of the cutting edge, which remains substantially constant or becomes slightly smaller starting from the region in the centre of rotation (h1a) as far as the region at the periphery of rotation (h2a), and a second stop surface (10b), which extends as far as the opening of the following cutting edge and delimits the penetration into the tissue by the cutting edge following the first penetration, has a second height difference (h1b, h2b) between the region located at the opening and the tip of the cutting edge, which is reduced progressively starting from the region of the centre of rotation (h1b) as far as the region at the periphery of rotation (h2b).

2. Surgical machining tool according to claim 1, with a groove-like depression (30) extending in a spiral on the convex surface (10), and which breaks through the cutting edges (20) on the convex surface in the region of the recesses (24) in the cutting edges in such manner that the groove-like depression (30) forms a practically continuously flush continuation of the recesses (24) in the cutting edges, and a consequently chip-breaking division of the cutting edges (20) into cutting elements is created, wherein the length of the cutting elements diminishes progressively in the direction starting from the centre of rotation (12) to the periphery of rotation (14).

3. Surgical machining tool according to claim 1 or 2, wherein the openings (22) are conformed continually open in a direction substantially parallel to the cutting edges (20) and terminate in the region of the centre of rotation (12) and in the region of the periphery of rotation (14).

4. Surgical machining tool according to any one of claims 1 to 3, **characterised in that** when viewed in cross-section the cutting edges (20) each have a rake angle alpha, which is formed between the convex surface (10) and a cutting surface (28) facing away from the workpiece, and which increases progressively from the cutting edge in the region of the centre of rotation toward the cutting edge in the region on the periphery of rotation.

5. Surgical machining tool according to any one of claims 1 to 4, **characterised in that** when viewed in cross-section the cutting edges (20) each have a clearance angle gamma, which is formed between the convex surface and the cutting surface (26) facing toward the workpiece, and which decreases progressively from the cutting edge in the region of the centre of rotation toward the cutting edge in the region on the periphery of rotation.

6. Surgical machining tool according to any one of claims 1 to 5, **characterised in that** the clearance angle gamma decreases in steps and/or constantly depending on the distance from the centre of rotation (12).

7. Surgical machining tool according to any one of claims 1 to 6, **characterised in that** the stop surfaces (10a, 10b) have a concave cross section.

8. Surgical machining tool according to any one of claims 1 to 7, **characterised in that** the cutting surface (26) of the tool that faces the workpiece remains substantially constant extending over the entire cutting edge (20).

9. Surgical machining tool according to any one of claims 1 to 8, **characterised in that** the cross section of the groove-shaped depression (30) is rounded or angular.

10. Surgical machining tool according to any one of claims 1 to 9, **characterised in that** the cutting edge is divided into about 5 to 8 cutting elements by recesses (24).

11. Surgical machining tool according to any one of claims 1 to 10, **characterized in that** the milling area is hemispherical or triangular or flat or tapered or conical or mushroom-shaped.

12. Surgical machining tool according to any one of claims 1 to 11, **characterised in that** the milling area has recesses in the side of the convex surface to enable the milling area to be brought close to areas of tissue that are hard to reach, wherein the shrouding of the milling area remains virtually unchanged compared to the full tool.

13. Surgical machining tool according to any one of claims 1 to 12, **characterised in that** the milling area comprises from about 2 to 8, preferably 6 cutting edges (20), preferably arranged at equal distances over the convex surface (10).

14. Surgical machining tool according to any one of claims 1 to 12, **characterized in that** the milling area is made from a highly heat-resistant metal, particularly a Cr-Co alloy.

## Revendications

1. Outil de traitement chirurgical destiné à la création d'un évidement dans un tissu corporel fixe, en particulier un os et/ou un cartilage, comprenant une partie de fraisage qui est rotative sur un axe de rotation central, comprenant une surface d'enveloppe (10) extérieure de la partie de fraisage et une partie intérieure (50) délimitée essentiellement par la surface d'enveloppe et éloignée du tissu corporel à traiter, sachant que la surface d'enveloppe est conçue avec au moins deux arêtes de coupe (20) par enlèvement de copeaux au sens d'une tête de fraisage, lesquelles passent, sur la surface d'enveloppe (10), d'un centre de rotation (12) à un bord de rotation (14) de la partie de fraisage et sont particulièrement formées en étant incurvées en spirale, et avec des ouvertures (22) disposées dans le voisinage des arêtes de coupe (20) pour évacuer les copeaux de coupe dans la partie intérieure (50), sachant que les arêtes de coupe (20) sont interrompues par des évidements de telle sorte que les arêtes de coupe sont composées d'éléments de coupe agissant en coupant individuellement,
**caractérisé en ce que** la surface d'enveloppe (10) est formée entre deux arêtes de coupe (20) d'au moins deux surfaces d'arrêt (10a, 10b) voisines, essentiellement parallèles aux arêtes de coupe (20), qui forment ensemble une ligne de séparation et qui délimitent la pénétration des arêtes de coupe (20) par rapport à la profondeur de pénétration dans le tissu, sachant que la ligne de séparation délimite une première surface d'arrêt (10a) qui est formée en étant détournée de l'arête de coupe et délimite une première pénétration de l'arête de coupe dans le tissu, et la ligne de séparation présente une première différence de hauteur (h1a, h2a) par rapport à une pointe d'arête de coupe, qui reste essentiellement constante ou diminue faiblement en partant de la zone dans le point de rotation (h1a) vers la zone sur le bord de rotation (h2a), et une seconde surface d'arrêt (10b) qui s'étend jusqu'à l'ouverture de l'arête de coupe suivante et délimite la pénétration, consécutive à la première pénétration, de l'arête de coupe dans le tissu, présente une seconde différence de hauteur (h1b, h2b) entre la zone se trouvant sur l'ouverture et la pointe de l'arête de coupe, qui diminue en partant de la zone dans le centre de rotation (h1b) vers la zone sur le bord de rotation (h2b).

2. Outil de traitement chirurgical selon la revendication 1, comprenant un évidement (30) en forme de rainure passant en spirale sur l'enveloppe extérieure (10), qui perce les arêtes de coupe (20) sur la surface d'enveloppe au niveau des évidements (24) dans les arêtes de coupe de telle façon que l'évidement en forme de rainure (30) forme un prolongement presque continuellement bord à bord des évidements (24) dans les arêtes de coupe et il en résulte une répartition des arêtes de coupe (20) en éléments de coupe, cassant les copeaux , sachant que la longueur des éléments de coupe diminue en partant de la zone dans le centre de rotation (2) vers la zone sur le bord de rotation (14).

3. Outil de traitement chirurgical selon la revendication 1 ou 2, dans lequel les ouvertures (22) sont formées essentiellement en étant ouvertes et parallèles aux arêtes de coupe (20) en les traversant et se terminent au niveau du centre de rotation (12) et au niveau du bord de rotation (14).

4. Outil de traitement chirurgical selon l'une des revendications 1 à 3, **caractérisé en ce que** les arêtes de coupe (20), vues en section, présentent respectivement un angle de dégagement alpha qui est formé entre la surface d'enveloppe (10) et une surface de coupe (28) éloignée de la pièce, et qui augmente de l'arête de coupe au niveau du centre de rotation jusqu'à l'arête de coupe au niveau du bord de rotation.

5. Outil de traitement chirurgical selon l'une des revendications 1 à 4, **caractérisé en ce que** les arêtes de coupe (20), vues en section, présentent respectivement un angle libre gamma qui est formé entre la surface d'enveloppe et la surface de coupe (26) tournée vers la pièce, et qui décroit de l'arête de coupe au niveau du centre de rotation jusqu'à l'arête de coupe au niveau du bord de rotation.

6. Outil de traitement chirurgical selon l'une des revendications 1 à 5, **caractérisé en ce que** l'angle libre gamma décroit progressivement et/ou continuellement en fonction de la distance par rapport au centre de rotation (12).

7. Outil de traitement chirurgical selon l'une des revendications 1 à 6, **caractérisé en ce que** les surfaces d'arrêt (10a, 10b) présentent une section concave.

8. Outil de traitement chirurgical selon l'une des revendications 1 à 7, **caractérisé en ce que** la surface de coupe (26) de l'outil tournée vers la pièce, reste essentiellement constante sur toute l'arête de coupe (20).

9. Outil de traitement chirurgical selon l'une des revendications 1 à 8, **caractérisé en ce que** la section de l'évidement (30) en forme de rainure est arrondie ou angulaire.

10. Outil de traitement chirurgical selon l'une des revendications 1 à 9, **caractérisé en ce que** l'arête de coupe est divisée en 5 à 8 éléments de coupe par des évidements (24).

11. Outil de traitement chirurgical selon l'une des revendications 1 à 10, **caractérisé en ce que** la partie de fraisage est semi-sphérique ou triangulaire ou planaire ou tronconique ou conique ou en forme de champignon.

12. Outil de traitement chirurgical selon l'une des revendications 1 à 11, **caractérisé en ce que** la partie de fraisage présente des évidements dans le côté de la surface d'enveloppe pour amener la partie de fraisage aux endroits difficilement accessibles du tissu, sachant que l'extrémité enveloppante de la partie de fraisage reste presque inchangée par rapport à la totalité de l'outil.

13. Outil de traitement chirurgical selon l'une des revendications 1 à 12, **caractérisé en ce que** la partie de fraisage présente environ 2 à 8, de préférence 6 arêtes de coupe, disposées de préférence à équidistance sur la surface d'enveloppe (10).

14. Outil de traitement chirurgical selon l'une des revendications 1 à 12, **caractérisé en ce que** la partie de fraisage est constituée d'un métal résistant aux hautes températures, en particulier d'un alliage Cr-Co.
